(19) **Europäisches Patentamt**
**European Patent Office**
**Office européen des brevets**

(11) **EP 4 458 894 A1**

(12) **EUROPEAN PATENT APPLICATION**
published in accordance with Art. 153(4) EPC

(43) Date of publication:
**06.11.2024 Bulletin 2024/45**

(21) Application number: **23781248.2**

(22) Date of filing: **24.03.2023**

(51) International Patent Classification (IPC):
*C08K 5/372* (2006.01)    *C08K 5/20* (2006.01)
*C08L 33/06* (2006.01)    *C08J 3/24* (2006.01)

(52) Cooperative Patent Classification (CPC):
**C08J 3/24; C08K 5/20; C08K 5/372; C08L 33/06**

(86) International application number:
**PCT/KR2023/003922**

(87) International publication number:
**WO 2023/191392 (05.10.2023 Gazette 2023/40)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **29.03.2022 KR 20220039062
23.03.2023 KR 20230038011**

(71) Applicant: **LG Chem, Ltd.**
**Yeongdeungpo-gu**
**Seoul 07336 (KR)**

(72) Inventors:
• **MIN, Ji Hong**
  **Daejeon 34122 (KR)**
• **KIM, Jun Kyu**
  **Daejeon 34122 (KR)**

(74) Representative: **Plasseraud IP**
**104 Rue de Richelieu**
**CS92104**
**75080 Paris Cedex 02 (FR)**

(54) **SUPERABSORBENT POLYMER COMPOSITION AND PREPARATION METHOD THEREFOR**

(57)    The present disclosure relates to a composition of super absorbent polymer and a preparation method thereof, and more specifically, a composition of super absorbent polymer and a preparation method thereof, that may improve deodorization capacity and odor masking capacity by using specific additives in combination, to effectively inhibit odor generated from urine, and the like, when applied to a product such as a diaper, and the like, and bacteria growth during wearing of a product.

**EP 4 458 894 A1**

**Description**

[Technical Field]

**Cross-reference to Related Applications**

This application claims the benefit of Korean Patent Application No. 10-2022-0039062 filed on March 29, 2022 and Korean Patent Application No. 10-2023-0038011 filed on March 23, 2023 with the Korean Intellectual Property Office, the disclosures of which are herein incorporated by reference in their entirety.

The present disclosure relates to a composition of super absorbent polymer and a preparation method thereof. More specifically, the present disclosure relates to a composition of super absorbent polymer and a preparation method thereof, that may improve deodorization capacity and odor masking capacity by using specific additives in combination, to effectively inhibit odor generated from urine, and the like, when applied to a product such as a diaper, and the like, and bacteria growth during wearing of a product.

[Background Art]

Super absorbent polymer (SAP) is synthetic polymer material that can absorb moisture of 500 to 1000 times of self-weight, and is also named differently as super absorbency material (SAM), absorbent gel material (AGM), etc. according to developing companies. The superabsorbent polymer began to be commercialized as sanitary items, and currently, it is being widely used as water-holding material for soil, water stop material for civil engineering and architecture, sheets for raising seedings, freshness preservatives in the field of food circulation, fomentation material, etc., besides hygienic goods such as a disposable diaper for children, and the like.

In most cases, such superabsorbent polymer is being widely used in the field of hygienic goods such as diapers or sanitary pad, and the like. In the hygienic goods, the superabsorbent polymer is generally spread in pulp and included. However, recently, to provide hygienic goods such as diapers having thinner thickness, development of diapers having decreased pulp content, or even pulpless diapers in which pulp is not used at all, is being actively progressed.

As such, in the case of hygienic goods having decreased pulp, or pulpless hygienic goods, superabsorbent polymer is included at relatively high ratio, and such superabsorbent polymer particles are included inevitably in multilayers. The superabsorbent polymer should basically exhibit high absorption performance and absorption speed so that the entire superabsorbent polymer particles included in multilayers can more efficiently absorb liquid such as urine. Moreover, absorbed liquid should not be leaked even under external pressure, and besides, the superabsorbent polymer should have permeability so as to properly maintain the original shape even when it absorbs liquid and is swollen. Thus, a lot of research is being progressed, such as surface crosslinking, for improving basic absorption force and water holding capacity.

Meanwhile, super absorbent polymer may be used in hygienic products, and in this case, due to the odor of absorbed liquids such as excretion of human and pet, afterfeel may be degraded. Further, bacteria growth is accelerated as the wearing time passes, thus generating odor.

Thus, demand for odor inhibition as well as basic properties of super absorbent polymer including absorption power and water holding capacity is gradually increasing. Thus, preparation of super absorbent polymer that has deodorization capacity and can effectively inhibit bacteria growth, is needed.

[Disclosure of Invention]

[Technical Problem]

It is an object of the invention to provide a composition of super absorbent polymer and a preparation method thereof. More specifically, it is an object of the invention to provide a composition of super absorbent polymer and a preparation method thereof, that improves deodorization capacity and odor masking capacity by using specific additives in combination, to effectively inhibit odor such as urine and bacteria growth when applied to a product such as a diaper, and the like.

[Technical Solution]

To solve the objects, according to the present disclosure, there is provided a composition of super absorbent polymer comprising:

　　super absorbent polymer comprising a base resin comprising crosslinked polymer formed by crosslinking polymerization of acrylic acid-based monomers having at least partially neutralized acid groups and an internal crosslinking agent, and a surface crosslink layer formed on the surface of the base resin, in which the crosslinked polymer is additionally crosslinked by a surface crosslinking agent;
an amino acetate-based chelating agent; and
cysteine,
wherein the amino acetate-based chelating agent and cysteine are each independently included inside the surface crosslink layer or on the surface of the surface crosslink layer.

According to the present disclosure, there is also provided a method for preparing a composition of super absorbent polymer comprising steps of:

subjecting acrylic acid-based monomers having at least partially neutralized acid groups to crosslinking polymerization, in the presence of an internal crosslinking agent and a polymerization initiator, to form hydrogel polymer (step 1);
preparing base resin comprising crosslinked polymer obtained by drying and grinding the hydrogel polymer (step 2);
mixing a surface crosslinking composition with the base resin to prepare a mixture (step 3); and
heat treating the mixture to prepare super absorbent polymer in which a surface crosslink layer is formed on the surface of the base resin (step 4),
wherein an amino acetate-based chelating agent and cysteine are each independently mixed in at least one steps of the steps 2 to 4 and steps before and after them.

[Effects]

As explained above, the present disclosure is characterized by providing a composition of super absorbent polymer that has excellent deodorization capacity by combined use of specific additives in super absorbent polymer, and inhibits bacteria growth to inhibit generation of additional odor, and a preparation method thereof.

[Best Mode for Carrying Out the Invention]

The terms used herein are only to explain specific embodiments, and are not intended to limit the invention.
A singular expression includes a plural expression thereof, unless the context clearly indicates otherwise. Throughout the specification, the terms "comprise", "equipped" or "have", etc. are intended to designate the existence of practiced characteristic, number, step, constructional element or combinations thereof, and they are not intended to preclude the possibility of existence or addition of one or more other characteristics, numbers, steps, constructional elements or combinations thereof.
The terms 'first', 'second', 'third' and the like are used to explain various constructional elements, and they are used only to distinguish one constructional element from the other constructional elements.
As used herein, the term "polymer" means a polymerized state of water soluble ethylenically unsaturated monomers, and may include polymers of all moisture content ranges or all particle diameter ranges. Among the polymers, polymer that is not dried and has a moisture content of about 40 wt% or more, may be referred to as hydrogel polymer, and ground and dried particles of such hydrogel polymer may be referred to as crosslinked polymer.
Further, as used herein, "base resin" or "base resin powder" means particle or powder obtained by drying and grinding polymer polymerized from acrylic acid-based monomers, before passing through surface modification or surface crosslinking step as described later.
Further, the term "super absorbent polymer" or "super absorbent polymer powder'" means crosslinked polymer polymerized from water soluble ethylenically unsaturated monomers including acid groups, at least a part of said acid groups being neutralized, or base resin powder consisting of super absorbent polymer particles obtained by grinding the crosslinked polymer, or it is used to include the crosslinked polymer or base resin made suitable for productization by subjecting it to additional processes, for example, surface crosslinking, fine powder reassembling, drying, grinding, classification, and the like.
Although various modifications can be made to the invention and the invention may have various forms, specific examples will be illustrated and explained in detail below. However, it should be understood that it is not intended to limit the invention to specific disclosure, and that the invention includes all the modifications, equivalents or replacements thereof without departing from the spirit and technical scope of the invention.
Hereinafter, a method for preparing super absorbent polymer and super absorbent polymer according to specific embodiments of the invention will be explained in detail.

**(Composition of super absorbent polymer)**

According to one embodiment of the invention, there is provided a composition of super absorbent polymer.

The super absorbent polymer comprises super absorbent polymer comprising a base resin comprising crosslinked polymer formed by crosslinking polymerization of acrylic acid-based monomers having at least partially neutralized acid groups and an internal crosslinking agent, and a surface crosslink layer formed on the surface of the base resin, in which the crosslinked polymer is additionally crosslinked by a surface crosslinking agent; an amino acetate-based chelating agent; and cysteine,
wherein the amino acetate-based chelating agent and cysteine are each independently included inside the surface crosslink layer or on the surface of the surface crosslink layer.

Super absorbent polymer is variously used in hygienic products such as diapers, sanitary pads, and the like, and in this case, due to the odor of excretion of human and pet, afterfeel may be degraded. Further, as the wearing time passes, bacteria growth is accelerated by the liquid absorbed in the product, thus generating additional odor.

Deodorant substances previously included in a product to reduce odor should be excessively used to realize deodorization capacity of an aimed degree, and thus, absorption properties may be significantly deteriorated, and production cost may increase.

Thus, the inventors of the present disclosure found out that by using specific additives in combination, odor generated by various causes may be effectively controlled without deterioration of basic properties of super absorbent polymer including absorption force and water holding capacity, and completed the invention.

Specifically, according to the present disclosure, both primary odor resulting from odor-generating substance included in body fluid, urine, and the like, when the product is applied, and secondary odor resulting from bacteria growth can be effectively controlled. Moreover, by combined use of two kinds of additives, excellent deodorization capacity and odor masking capacity can be realized with relatively small amount, which is economical, and particularly, does not reduce absorption properties.

The amino acetate-based chelating agent is a component capable of effectively inhibiting the growth of bacteria such as E. coli caused by odor-generating substance. As the wearing time of a product passes, bacteria growth is accelerated by the odor-generating substance remaining in the product, and thus, additional odor is generated, but the amino acetate-based chelating agent inhibits such bacteria growth to effectively reduce additional odor generation.

Particularly, the amino acetate-based chelating agent is a divalent cation forming a salt crosslink between the constituents of cell membrane, and it can effectively inhibit bacteria growth by destructing the cell membrane of bacteria.

The amino acetate-based chelating agent may include, for example, one or more selected from the group consisting of ethylenediamine tetraacetic acid (EDTA), L-glutamic acid diacetate (GLDA), methyl glycine diacetic acid (MGDA), hydroxyethyl ethylenediamine triacetic acid (HEDTA), ethanol diglycinic acid (EDG), diethylenetriamine pentaacetic acid (DTPA), and salts thereof.

The amino acetate-based chelating agent may be included in the content of 0.01 to 3 parts by weight, based on 100 parts by weight of the base resin, and preferably, may be included in the content of 0.025 parts by weight or more, 0.05 parts by weight or more, 0.1 parts by weight or more, 2.5 parts by weight or less, 2.0 parts by weight or less, 1.5 parts by weight or less, or 0.01 to 2.5 parts by weight, 0.01 to 2.0 parts by weight, 0.025 to 3.0 parts by weight, 0.025 to 2.5 parts by weight, 0.05 to 2.5 parts by weight, 0.05 to 2.0 parts by weight, 0.1 to 2.5 parts by weight, or 0.1 to 2.0 parts by weight.

If used in the above content range, it can effectively inhibit bacteria without deterioration of absorption properties, and thus, remarkably improve deodorization capacity and odor masking capacity of absorbent polymer. The chelating agent may be mixed and used in the form of a salt with an aqueous solution, and thus, the content range is based on the solid content. Meanwhile, in case the chelating agent is included in a too small amount, bacteria inhibition property of an aimed degree may not be realized, and in case included in an excessive amount, unique properties of super absorbent polymer may be deteriorated.

The cysteine may chemically react with odor-generating substance to reduce odor. The odor-generating substance generally has small molecular weight, and it reacts with cysteine, and thereby, odor may be reduced or removed, and thus, effectively inhibited. Particularly, cysteine is effective for reducing odor generated from aldehydes and ketones. Meanwhile, in case similar amino acid methionine, and the like, are used, deodorization capacity may be exhibited to some degree, but they have different main functional groups, and thus, even if applied to SAP in the same amount, absorption properties and deodorization capacity may be remarkably deteriorated, compared to cysteine.

The cysteine may be included in the content of 0.01 to 3 parts by weight, based on 100 parts by weight of the based resin, and preferably, it may be included in the content of 0.025 parts by weight or more, 0.05 parts by weight or more, 0.1 parts by weight or more, 2.5 parts by weight or less, 2.0 parts by weight or less, 1.5 parts by weight or less, or 0.01 to 2.5 parts by weight, 0.01 to 2.0 parts by weight, 0.025 to 3.0 parts by weight, 0.025 to 2.5 parts by weight, 0.05 to 2.5 parts by weight, 0.05 to 2.0 parts by weight, 0.1 to 2.5 parts by weight, or 0.1 to 2.0 parts by weight.

In case used in the above content range, it may effectively inhibit odor generation without deteriorating absorption properties, thus remarkably improving deodorization capacity and odor masking capacity of absorbent polymer. The cysteine may be mixed and used in the form of a salt with an aqueous solution, and thus, the content range is based on the solid content. Mean while, if the cysteine is included in a too small amount, deodorization property may not be realized, and if included in an excessive amount, unique properties of super absorbent polymer may be deteriorated, and there may be process problem due to incomplete dissolving.

The base resin comprises crosslinked polymer formed by crosslinking polymerization of acrylic acid-based monomers having at least partially neutralized acid groups and an internal crosslinking agent

The acrylic acid-based monomers may be any monomers commonly used in the preparation of super absorbent polymer. As non-limiting examples, the acrylic acid-based monomer may be a compound represented by the following Chemical Formula 1:

[Chemical Formula 1]    $R^1\text{-}COOM^1$

In the Chemical Formula 1,

$R^1$ is a C2-5 alkyl group comprising an unsaturated bond,
$M^1$ is a hydrogen atom, a monovalent or divalent metal, an ammonium group or an organic amine salt.

Preferably, the monomers may be one or more selected from the group consisting of acrylic acid, methacrylic acid, and monovalent metal salts, divalent metal salts, ammonium salts and organic amine salts thereof.

The acrylic acid-based monomers may have acid groups, and at least a part of the acid groups may be neutralized. Preferably, the monomers partially neutralized with alkali substance such as sodium hydroxide, potassium hydroxide, ammonium hydroxide and the like, may be used.

Wherein, the degree of neutralization of the monomers may be 40 to 95 mol%, or 40 to 80 mol%, or 45 to 75 mol%. Although the range of the neutralization degree may vary according to the final properties, if the neutralization degree is too high, neutralized monomers may be precipitated, and thus, it may be difficult to smoothly progress polymerization, and to the contrary, if the neutralization degree is too low, absorption force of polymer may be significantly lowered, and it may exhibit elastic rubber-like properties, which is difficult to handle.

The "internal crosslinking agent" is used to distinguish it from a "surface crosslinking agent" for crosslinking the surface of base resin, and it functions for crosslinking the unsaturated bonds of the above-explained acryl-based monomers to polymerize. Crosslinking in this step progresses without distinction of the surface or inside, but by the surface crosslinking process of base resin as described later, the surface of the finally prepared super absorbent polymer particle consists of a structure crosslinked by the surface crosslinking agent, and the inside consists of a structure crosslinked by the internal crosslinking agent.

As the internal crosslinking agent, any compounds can be used so long as they enable the introduction of crosslink during polymerization of the acrylic acid-based monomers. As non-limiting examples, as the internal crosslinking agent, multifunctional crosslinking agents such as N,N'-methylenebisacrylamide, trimethylolpropane tri(meth)acrylate, ethyleneglycol di(meth)acrylate, polyethyleneglycol (meth)acrylate, propyleneglycol di(meth)acrylate, polypropyleneglycol (meth)acrylate, butanediol di(meth)acrylate, butyleneglycol di(meth)acrylate, diethyleneglycol di(meth)acrylate, hexanediol di(meth)acrylate, triethyleneglycol di(meth)acrylate, tripropyleneglycol di(meth)acrylate, tetraethyleneglycol di(meth)acrylate, dipentaerythritol pentaacrylate, glycerin tri(meth)acrylate, pentaerythritol tetraacrylate, triallylamine, ethyleneglycol diglycidyl ether, propyleneglycol, glycerin, or ethylenecarbonate may be used alone or in combinations, but it is not limited thereto.

Such an internal crosslinking agent may be used at the concentration of 0.001 to 1 wt%, or 0.01 to 0.8 wt%, or 0.1 to 0.7 wt%, based on the monomer composition. If the concentration of the internal crosslinking agent is too low, absorption speed of polymer may decrease, and gel strength may become weak. To the contrary, if the concentration of the internal crosslinking agent is too high, absorption force of superabsorbent polymer may decrease, and thus, it may not be preferable as an absorbent.

Besides, the monomer composition may further comprise additives such as a thickener, a plasticizer, a preservation stabilizer, an antioxidant, etc., as necessary.

The super absorbent polymer comprises a surface crosslink layer formed on the surface of the base resin, in which the crosslinked polymer is additionally crosslinked by a surface crosslinking agent

Wherein, the amino acetate-based chelating agent and cysteine are each independently, included inside the surface crosslink layer or on the surface of the surface crosslink layer. It means that the amino acetate-based chelating agent and cysteine are each independently added after polymerization of base resin (= before a surface crosslinking step), during the surface crosslinking step, or after the surface crosslinking step, which will be explained in detail in the preparation method of a composition of super absorbent polymer as described later.

The surface crosslink layer is formed by additional crosslinking of the crosslinked polymer by a surface crosslinking agent, wherein the surface crosslinking agent is not specifically limited so long as it is commonly used for surface crosslinking of super absorbent polymer and is a compound capable of reacting with the functional groups of the polymer.

Preferably, to improve the properties of produced super absorbent polymer, as the surface crosslinking agent, one or more selected from the group consisting of polyhydric alcohol compounds; epoxy compounds; polyamine compounds; haloepoxy compounds; condensation products of haloepoxy compounds; oxazoline compounds; mono-, di- or polyoxazolidinone compounds; cyclic urea compounds; multivalent metal salts; and alkylene carbonate compounds may be used.

Specifically, as the examples of the polyhydric alcohol compound, one or more selected from the group consisting of mono-, di-, tri- tetra- or polyethylene glycol, monopropylene glycol, 1,3-propanediol, dipropylene glycol, 2,3,4-trimethyl-1,3-pentanediol, polypropylene glycol, glycerol, polyglycerol, 2-butene-1,4-diol, 1,4-butanediol, 1,3-butanediol, 1,5-pentanediol, 1,6-hexandiol, and 1,2-cyclohexanedimethanol may be used.

Further, as the epoxy compound, ethylene glycol diglycidyl ether and glycidol, and the like may be used, and as the polyamine compound, one or more selected from the group consisting of ethylenediamine, diethylenetriamine, triethylenetriamine, tetraethylenepentamine, pentaethylenehexamine, polyethyleneamine, and polyamidepolyamine may be used.

Further, as the haloepoxy compound, epichlorohydrin, epibromohydrin, and $\alpha$-methylepichlorohydrin may be used, and as the mono-, di- or polyoxazolidinone compound, for example, 2-oxazolidinone, and the like may be used.

Further, as the alkylene carbonate compound, ethylene carbonate, and the like may be used. These compounds may be used alone or in combinations. Meanwhile, to increase the efficiency of the surface crosslinking process, among these surface crosslinking agents, one or more kinds of C2-10 polyhydric alcohol compounds may be included.

The content of the surface crosslinking agent added may be appropriately selected according to the kind of the surface crosslinking agent added and reaction conditions, but commonly, it may be used in the amount of about 0.001 to about 5 parts by weight, preferably about 0.01 to about 3 parts by weight, more preferably about 0.05 to about 2 parts by weight, based on 100 parts by weight of the polymer.

If the content of the surface crosslinking agent is too small, a surface crosslinking reaction may hardly occur, and if it is greater than 5 parts by weight, based on 100 parts by weight of the polymer, due to the excessive progression of surface crosslinking reaction, absorption capacity and properties may be deteriorated.

Meanwhile, the surface crosslinking agent may further comprise inorganic materials. As such inorganic material, one or more selected from the group consisting of silica, clay, alumina, silica-alumina composite material, titania, zinc oxide and aluminum sulfate may be used. The inorganic material may be used in the form of powder or liquid, and particularly, alumina powder, silica-alumina powder, titania powder, or a nano silica solution. The inorganic material may be used in the content of about 0.001 to about 1 part by weight, based on 100 parts by weight of the base resin.

Further, the surface crosslinking agent may further comprise a thickener. If the surface of base resin powder is additionally crosslinked in the presence of a thickener, property deterioration may be minimized even after grinding. Specifically, as the thickener, one or more selected from polysaccharide and hydroxy-containing polymer may be used. As the polysaccharide, a gum-based thickener and a cellulose-based thickener, and the like may be used. As specific examples of the gum-based thickener, xanthan gum, arabic gum, karaya gum, tragacanth gum, ghatti gum, guar gum, locust bean gum and psyllium seed gum, and the like may be mentioned, and as specific examples of the cellulose-based thickener, hyrdoxypropylmethylcellulose, carboxymethylcellulose, methylcellulose, hydroxymethylcellulose, hydroxyethylcellulose, hydroxypropylcellulose, hydroxyethylmethylcellulose, hydroxymethylpropylcellulose, hydroxyethylhydroxypropylcellulose, ethylhydroxyethylcellulose and methylhydroxypropylcellulose, and the like may be mentioned. Meanwhile, as specific examples of the hydroxy-containing polymer, polyethyleneglycol and polyvinylalcohol, and the like may be mentioned.

According to another embodiment of the invention, the composition of super absorbent polymer may further comprise one or more additives selected from the group consisting of metal iodide salt, organic acid and polyphenol, and by the additives, deodorization capacity and odor masking capacity may be further improved.

The metal iodide salt may be, for example, in the form wherein one or more selected from the group consisting of CuI, NaI and KI, and $I_2$ are dissolved in water together. The metal iodide salt may oxidize malodorous substance to confer deodorization property.

The organic acid may be, for example, one or more selected from the group consisting of citric acid, glutamic acid, ascorbic acid, benzoic acid and erythorbic acid.

The polyphenol may be, for example, one or more selected from the group consisting of tannin, anthocyanin, flavonol, isoflavon, catechin, polyquercetin, and caffeic acid.

The additives may be each independently included in the content of, preferably, 0.025 parts by weight or more, 0.05 parts by weight or more, 0.1 parts by weight or more, 2.5 parts by weight or less, 2.0 parts by weight or less, 1.5 parts by weight or less, or 0.01 to 2.5 parts by weight, 0.01 to 2.0 parts by weight, 0.025 to 2.5 parts by weight, 0.05 to 2.5 parts by weight, 0.05 to 2.0 parts by weight, 0.1 to 2.5 parts by weight, or 0.1 to 2.0 parts by weight, based on 100 parts by weight of the base resin.

If used in the above content range, they can realize synergistic effect with cysteine.

Meanwhile, in case the metal iodide salt is included in an excessive amount, unique properties of super absorbent polymer may be deteriorated, and it may be fed as a solution during the process to increase tailings, thus decreasing deodorization capacity to the contrary. The metal iodide salt is mixed with super absorbent polymer in the state of an aqueous solution with water as a solvent, and exists in the super absorbent polymer while being mixed and physically contained in the super absorbent polymer. The metal iodide salt may be a solution with a solid content concentration of 1 % or less.

Meanwhile, the additives may be each independently included inside the surface crosslink layer or on the surface of the surface crosslink layer. It means that each additive is added during polymerization of base resin (=before the surface crosslinking step), in the surface crosslinking step, or after the surface crosslinking step, which will be explained in detail in the preparation method of a composition of super absorbent polymer as described later.

**(Preparation method of composition of super absorbent polymer)**

According to one embodiment of the invention, there is provided a method for preparing a composition of super absorbent polymer.

The method for preparing a composition of superabsorbent polymer comprises steps of:
subjecting acrylic acid-based monomers having at least partially neutralized acid groups to crosslinking polymerization, in the presence of an internal crosslinking agent and a polymerization initiator, to form hydrogel polymer (step 1); preparing base resin comprising crosslinked polymer obtained by drying and grinding the hydrogel polymer (step 2); mixing a surface crosslinking composition with the base resin to prepare a mixture (step 3); and heat treating the mixture to prepare super absorbent polymer in which a surface crosslink layer is formed on the surface of the base resin (step 4), wherein an amino acetate-based chelating agent and cysteine are each independently mixed in at least one steps of the steps 2 to 4 and steps before and after these steps.

The preparation method of a composition of super absorbent polymer can effectively control odor generated by various causes, without deteriorating basic properties of super absorbent polymer including absorption force and water holding capacity, by using an amino acetate-based chelating agent and cysteine in combination. To the amino acetate-based chelating agent and cysteine, the foregoing may be applied identically.

Meanwhile, the expression 'mixed in step A' means to be additionally mixed with a target mixture while the step A is conducted, and it may mean to be divided more than once and mixed at the content ratio aimed in corresponding step. Meanwhile, the expression 'mixed before and after step A' means to be additionally mixed before the step A is conducted or after the step A is finished.

More specifically, the amino acetate-based chelating agent and cysteine are each independently mixed in at least one step of the steps 2 to 4 and steps before and after these steps, which means that they are mixed during the surface crosslinking step, before the surface crosslinking step, or after the surface crosslinking step is finished, and they may be mixed in one or more steps. The two kinds of additives are included inside the surface crosslink layer or on the surface of the surface crosslink layer, and thereby, react with odor-generating substance and have excellent deodorization capacity, and inhibit bacteria growth to effectively inhibit additional odor generation. Meanwhile, in case the two kinds of additives are mixed in the polymerization step, it may be difficult to realize an aimed effect.

More preferably, the amino acetate-based chelating agent may be mixed in the step 2, or mixed after the step 4. More specifically, in case the chelating agent is included in the step of preparing base resin from polymerized hydrogel polymer (step 2), it means that it is be mixed with base resin and is added and mixed in the drying and grinding step. In this case, the chelating agent may be included inside the surface cross link layer formed in the surface crosslinking step. Further, in case the chelating agent is mixed after the surface crosslinking step (step 4), it means that it is mixed with super absorbent polymer having a surface crosslink layer. In this case, the chelating agent may be included on the surface of the surface crosslink layer formed in the surface crosslinking step.

More preferably, the cysteine may be mixed after the step 4. Specifically, in case the cysteine is mixed after the surface crosslinking step (step 4), it means that it is mixed with super absorbent polymer having a surface crosslink layer. In this case, the chelating agent may be included on the surface of the surface crosslink layer formed in the surface crosslinking step. In case the cysteine is included after the surface crosslinking step (step 4), it exists on the surface of super absorbent polymer, and thus, the contact area with odor may increase, which may be favorable in terms of improvement in deodorization capacity.

According to one embodiment of the invention, in the preparation method of super absorbent polymer, one or more additives selected from the group consisting of metal iodide salt, organic acid and polyphenol may be further included, and to the additives, the foregoing may be applied identically.

The additives may be mixed in at least one step of the steps 2 to 4 and steps before and after these steps, which means that they are mixed during the surface crosslinking step, before the surface crosslinking step, or after the surface crosslinking step is finished, and they may be mixed in one or more steps. Thereby, the additives are included inside the surface crosslink layer or on the surface of the surface crosslink layer, and can more effectively inhibit odor generation.

More preferably, in case the additives are included after the surface crosslinking step (step 4), it means that they are mixed with super absorbent polymer having a surface crosslink layer. In this case, the additives exist on the surface of the surface crosslink layer formed in the surface crosslinking step, and thus, the contact area with odor increases, which may be favorable in terms of improvement in deodorization capacity.

Hereinafter, the invention will be explained in detail according to steps.

**(Step 1)**

The step 1 is a step of preparing hydrogel polymer, and specifically, a step wherein a monomer composition comprising acrylic acid-based monomers having at least partially neutralized acid groups is subjected to crosslinking polymerization to form hydrogel polymer.

The acrylic acid-based monomers may be any monomers commonly used in the preparation of superabsorbent polymer. Specifically, to the acrylic acid-based monomers, the foregoing may be applied identically.

Further, in the monomer composition, a polymerization initiator commonly used for the preparation of super absorbent polymer may be included.

As the polymerization initiator, a thermal polymerization initiator or a photopolymeirzation initiator may be used according to polymerization method. However, even when photopolymerization is progressed, since a certain amount of heat is generated by UV irradiation, etc., and heat is generated to some degree according to the progression of an exothermic polymerization reaction, a thermal polymerization initiator may be additionally included.

As the photopolymerization initiator, one or more selected from the group consisting of benzoin ether, dialkyl acetophenone, hydroxyl alkylketone, phenyl glyoxylate, benzyl dimethyl Ketal, acyl phosphine, and $\alpha$-aminoketone may be used. Among them, as specific examples of acyl phosphine, commercially available lucirin TPO, i.e., 2,4,6-trimethyl-benzoyl-trimethyl phosphine oxide may be used. More various photopolymerization initiators are described in Reinhold Schwalm, "UV Coatings: Basics, Recent Developments and New Application(Elsevier 2007)", page 115, which may be referred to.

And, as the thermal polymerization initiator, one or more selected from the group consisting of a persulfate initiator, an azo initiator, hydrogen peroxide, and ascorbic acid may be used. Specific examples of the persulfate initiator may include sodium persulfate ($Na_2S_2O_8$), potassium persulfate ($K_2S_2O_8$), ammonium persulfate (($NH_4$)$_2S_2O_8$), etc., and, specific examples of the azo initiator may include 2,2-azobis(2-amidinopropane)dihydrochloride, 2,2-azobis-(N,N-dimethylene)isobutyramidinedihydrochloride, 2-(carbamoylazo)isobutyronitril, 2,2-azobis[2-(2-imidazolin-2-yl)propane] dihydrochloride, 4,4-azobis-(4-cyanovalericacid), etc. More various thermal initiators are described in "Principle of Polymerization (Wiley, 1981)", Odian, page 203, which may be referred to.

Such a polymerization initiator may be added at the concentration of 0.001 to 1 wt%, or 0.005 to 0.1 wt%, based on the monomer composition. If the concentration of the polymerization initiator is too low, polymerization speed may become slow, and remaining monomers may be extracted in a large quantity in the final product. And, if the concentration of the polymerization initiator is too high, polymer chains making up the network of superabsorbent polymer may become short, and thus, extractable contents may increase, and absorbency under pressure of superabsorbent polymer may be lowered, thereby deteriorating the properties of polymer.

Meanwhile, the polymerization of the monomer composition is conducted in the presence of an internal crosslinking agent to improve the properties of polymer obtained by the polymerization of the acrylic acid-based monomers. To the internal crosslinking agent, the foregoing may be applied identically.

Further, the crosslinking polymerization of the monomer composition may be conducted in the presence of a blowing agent, according to the necessity and degree of improvement in absorption speed. Such a blowing agent may be decomposed during the crosslinking polymerization reaction and generate gas, thus forming pores in the hydrogel polymer. As the result, when such a blowing agent is additionally used, more developed porous structure is formed in super absorbent polymer, and thus, absorption speed of super absorbent polymer may be further improved.

As non-limiting examples, the blowing agent may comprise one or more compounds selected from the group consisting of sodium bicarbonate, sodium carbonate, potassium bicarbonate, potassium carbonate, calcium bicarbonate, calcium carbonate, magnesium bicarbonate, magnesium carbonate, azodicarbonamide (ADCA), dinitroso pentamethylene tetramine (DPT), p,p'-oxybis(benzenesulfonyl hydrazide) (OBSH), p-toluenesulfonyl hydrazide (TSH), sucrose stearate, sucrose palmitate, and sucrose laurate.

The blowing agent may be included in the monomer composition in the content of 1000 to 4000 ppmw, and more specifically, in the content of 1000 ppm or more, or 1100 ppmw or more, or 1200 ppmw or more; and 4000 ppmw or less, or 3500 ppmw or less, or 3000 ppmw or less.

Besides, the monomer composition may further comprise a thickener, a plasticizer, a preservation stabilizer, an antioxidant, and the like, as necessary.

Further, the monomer composition may be prepared in the form of a solution in which the above-explained raw materials including acrylic acid-based monomers, polymerization initiator, internal crosslinking agent, blowing agent, and the like are dissolved in a solvent.

Wherein, the solvent that can be used is not limited so long as it can dissolve the above explained raw materials. For example, as the solvent, water, ethanol, ethyleneglycol, diethyleneglycol, triethyleneglycol, 1,4-butanediol, propyleneglycol, ethyleneglycol monobutyl ether, propyleneglycol monomethyl ether, propyleneglycol monomethyl ether acetate, methylethylketone, acetone, methylamylketone, cyclohexanone, cyclopentanone, diethyleneglycol monomethyl ether, diethyleneglycol ethyl ether, toluene, xylene, butyrolactone, carbitol, methylcellosolve acetate, N,N-dimethylacetamide, or a mixture thereof may be used.

The formation of hydrogel polymer through the polymerization of the monomer composition may be conducted by a common polymerization method, and the process is not specifically limited.

As non-limiting examples, the polymerization method is largely classified into thermal polymerization and photopolymerization according to the kind of energy source, and thermal polymerization may be progressed in a reactor equipped with a stirring axis such as a kneader, and photopolymerization may be progressed in a reactor equipped with a movable conveyer belt.

For example, hydrogel polymer may be obtained by adding the above- described monomer composition into a reactor such as a kneader equipped with a stirring axis, and supplying hot air or heating the reactor to progress thermal polymerization. Wherein, the hydrogel polymer discharged to the outlet of the reactor may be in the size of a few centimeters to a few millimeters according to the shape of the stirring axis equipped in the reactor. Specifically, the size of obtained hydrogel polymer may vary according to the concentration of the added monomer composition and the addition speed, etc., and commonly, hydrogel polymer with particle diameter of 2 to 50 mm may be obtained.

For another example, in case photopolymerization of the monomer composition is progressed in a reactor equipped with a movable conveyer belt as explained above, hydrogel polymer in the form of a sheet may be obtained. Wherein, the thickness of the sheet may vary according to the concentration of the added monomer composition and the addition speed, but commonly, it is preferable that the thickness of the sheet is controlled to 0.5 to 10 cm, to enable uniform polymerization of the entire sheet, and simultaneously, secure production speed, and the like.

Hydrogel polymer thus obtained may have a moisture content of 40 to 80 wt%. Wherein, the "moisture content" is the content of moisture occupied based on the total weight of hydrogel polymer, and it means a value obtained by subtracting the weight of polymer of a dry state from the weight of hydrogel polymer. Specifically, it is defined as a value calculated by measuring the weight loss according to moisture evaporation in the polymer while raising the temperature of polymer through infrared heating to dry. Wherein, the temperature is raised from room temperature to about 180ºC and then maintained at 180ºC, and the total drying time may be set to 20 minutes including a temperature raising step of 5 minutes.

**(Step 2)**

The step 2 of the present disclosure is a step of drying and grinding the hydrogel polymer prepared in step 1, to form base resin.

Specifically, to increase drying efficiency of hydrogel polymer and affect the morphology of super absorbent polymer, thus affecting various properties of super absorbent polymer including absorption speed, particularly to improve absorption speed of super absorbent polymer, in the present disclosure, a step of coarse grinding may be further included before drying the hydrogel polymer. Hereinafter, to distinguish it from grinding after drying, the grinding before drying is referred to as 'coarse grinding' herein for convenience.

The grinder used for the grinding is not limited, but specifically, it may include anyone selected from the group consisting of vertical pulverizer, turbo cutter, turbo grinder, rotary cutter mill, cutter mill, disc mill, shred crusher, crusher, chopper and disc cutter, but is not limited thereto.

Wherein, the coarse grinding step may be conducted such that the particle diameter of hydrogel polymer may become about 2 mm to about 10 mm. Grinding to particle diameter less than 2 mm would not be technically easy due to high moisture content of hydrogel polymer, and it may cause aggregation between ground particles. Meanwhile, if grinding to particle diameter greater than 10 mm, the effect for increasing the efficiency of the subsequent drying step may be insignificant.

The drying may be conducted at a temperature of 120 to 250ºC, 140 to 200ºC, or 150 to 190ºC. Wherein, the drying temperature may be defined as the temperature of a heat transfer medium supplied for drying or the temperature inside a drying reactor containing a heat transfer medium and polymer in the drying process. If the drying temperature is too low and drying time is lengthened, process efficiency may be deteriorated, and thus, to prevent the same, the drying temperature is preferably 120ºC or more. And, if the drying temperature is higher than needed, the surface of hydrogel polymer may be excessively dried, and thus, generation of fine powders may increase in the subsequent grinding step, and the properties of the final polymer may be deteriorated, and thus, to prevent the same, the drying

temperature is preferably 250°C or less.

Wherein, the drying time in the drying step is not specifically limited, but considering process efficiency and properties of polymer, and the like, it may be controlled to 20 minutes to 90 minutes under the above-described drying temperature.

The drying may be conducted using a common medium, and for example, the drying may be conducted by hot air supply, infrared irradiation, microwave irradiation or UV irradiation to the ground hydrogel polymer, and the like.

Further, preferably, drying is conducted such that dried polymer may have a moisture content of 0.1 to 10 wt%. Namely, in case the moisture content of dried polymer is less than 0.1 wt%, due to excessive drying, production cost may increase and crosslinked polymer may be degraded. And, if the moisture content of dried polymer is greater than 10 wt%, defects may be generated in the subsequent process.

Subsequently, the dried hydrogel polymer may be ground. It is a step for optimizing the surface area of base resin powder and super absorbent polymer. The grinding may be conducted such that the particle diameter of ground polymer may become 150 to 850 μm.

Wherein, as a grinder, common grinders such as a pin mill, a hammer mill, a screw mill, a roll mill, a disc mill, a jog mill, and the like may be used.

Further, to manage the properties of the finally productized super absorbent polymer, a step of selectively classifying particles having particle diameter range of 150 to 850 μm in the polymer particles obtained through the grinding step, is conducted.

Passing through the classification step, base resin may be obtained. Such base resin may have particle diameter of 150 to 850 μm, and may comprise fine powders with particle diameter less than 150 μm in the content of 2 wt% or less, or 1 wt% or less.

Meanwhile, as explained above, the amino acetate-based chelating agent may be included in the step of preparing base resin from polymerized hydrogel polymer (step 2), and in this case, aggregation of base resin may be prevented, and thus, property deterioration may be inhibited and the chelating agent may be distributed more uniformly. Meanwhile, the mixing method of the amino acetate-based chelating agent is not specifically limited, and it may be mixed by simply mixing, or spraying in the state of an aqueous solution.

**(Step 3 and Step 4)**

The step 3 of the present disclosure is a step of mixing the base resin prepared in the step 2 with a surface crosslinking composition, and the step 4 is a step of heat treating the mixture to prepare super absorbent polymer in which a surface crosslink layer is formed on the surface of the base resin.

The surface crosslinking composition used in step 3 comprises a surface crosslinking agent, and the surface crosslinking agent is not specifically limited so long as it is commonly used for surface crosslinking of super absorbent polymer and is a compound capable of reacting with the functional groups of the polymer.

Preferably, to improve the properties of produced super absorbent polymer, as the surface crosslinking agent, one or more selected from the group consisting of polyhydric alcohol compounds; epoxy compounds; polyamine compounds; haloepoxy compounds; condensation products of haloepoxy compounds; oxazoline compounds; mono-, di- or polyoxazolidinone compounds; cyclic urea compounds; multivalent metal salts; and alkylene carbonate compounds may be used.

Specifically, as the examples of the polyhydric alcohol compound, one or more selected from the group consisting of mono-, di-, tri- tetra- or polyethylene glycol, monopropylene glycol, 1,3-propanediol, dipropylene glycol, 2,3,4-trimethyl-1,3-pentanediol, polypropylene glycol, glycerol, polyglycerol, 2-butene-1,4-diol, 1,4-butanediol, 1,3-butanediol, 1,5-pentanediol, 1,6-hexanediol, and 1,2-cyclohexanedimethanol may be used.

Further, as the epoxy compound, ethylene glycol diglycidyl ether and glycidol, and the like may be used, and as the polyamine compound, one or more selected from the group consisting of ethylenediamine, diethylenetriamine, triethylenetriamine, tetraethylenepentamine, pentaethylenehexamine, polyethyleneamine, and polyamidepolyamine may be used.

Further, as the haloepoxy compound, epichlorohydrin, epibromohydrin, and $\alpha$-methylepichlorohydrin may be used, and as the mono-, di- or polyoxazolidinone compound, for example, 2-oxazolidinone, and the like may be used.

Further, as the alkylene carbonate compound, ethylene carbonate, and the like may be used. These compounds may be used alone or in combinations. Meanwhile, to increase the efficiency of the surface crosslinking process, among these surface crosslinking agents, one or more kinds of C2-10 polyhydric alcohol compounds may be included.

The content of the surface crosslinking agent added may be appropriately selected according to the kind of the surface crosslinking agent added and reaction conditions, but commonly, it may be used in the amount of about 0.001 to about 5 parts by weight, preferably about 0.01 to about 3 parts by weight, more preferably about 0.05 to about 2 parts by weight, based on 100 parts by weight of the polymer.

If the content of the surface crosslinking agent is too small, a surface crosslinking reaction may hardly occur, and if it is greater than 5 parts by weight, based on 100 parts by weight of the polymer, due to the progression of excessive surface crosslinking reaction, absorption capacity and properties may be deteriorated.

Meanwhile, the step of forming a surface crosslink layer may be conducted while the surface crosslinking composition further comprises inorganic materials. As such inorganic material, one or more selected from the group consisting of silica, clay, alumina, silica-alumina composite material, titania, zinc oxide and aluminum sulfate may be used. The inorganic material may be used in the form of powder or liquid, and particularly, alumina powder, silica-alumina powder, titania powder, or a nano silica solution. And, the inorganic material may be used in the content of about 0.001 to about 1 part by weight, based on 100 parts by weight of the base resin.

Further, the surface crosslinking composition may further comprise a thickener. If the surface of base resin powder is additionally crosslinked in the presence of a thickener, property deterioration may be minimized even after grinding. Specifically, as the thickener, one or more selected from polysaccharide and hydroxy-containing polymer may be used. As the polysaccharide, gum-based thickener and cellulose-based thickener, and the like may be used. As specific examples of the gum-based thickener, xanthan gum, arabic gum, karaya gum, tragacanth gum, ghatti gum, guar gum, locust bean gum and psyllium seed gum, and the like may be mentioned, and as specific examples of the cellulose-based thickener, hyrdoxypropylmethylcellulose, carboxymethylcellulose, methylcellulose, hydroxymethylcellulose, hydroxyethylcellulose, hydroxypropylcellulose, hydroxyethylmethylcellulose, hydroxymethylpropylcellulose, hydroxyethylhydroxypropylcellulose, ethylhydroxyethylcellulose and methylhydroxypropylcellulose, and the like may be mentioned. Meanwhile, as specific examples of the hydroxy-containing polymer, polyethyleneglycol and polyvinylalcohol, and the like may be mentioned.

Meanwhile, a method for mixing the base resin with the surface crosslinking composition may be appropriately selected without specific limitations, so long as it can uniformly mix them.

For example, a method of putting the surface crosslinking composition and the base resin in a reactor and mixing them, a method of spraying the surface crosslinking composition to the surface of the base resin, a method of continuously feeding the base resin and the surface crosslinking composition to a continuously operated mixer and mixing, and the like, may be used.

Wherein, the surface crosslinking composition may be a solution, and in case the solid content in the solution is 1 wt% or more, 3 wt% or more, 5 wt% or more, 10 wt% or more, and 50 wt% or less, 30 wt% or less, or 20 wt% or less, it may be appropriate to uniformly disperse in the base resin, and simultaneously agglomeration of the base resin may be prevented.

Next, the step 4 is a step of heat treating the mixture to prepare super absorbent polymer in which a surface crosslink layer is formed on the surface of the base resin, and specifically, the base resin and a surface crosslinking composition are heat treated to form an interpenetrating polymer network on the surface of the crosslinked polymer included in the base resin, thereby further improving the properties of super absorbent polymer. Through such surface modification, on the surface of the ground base resin particles, a surface crosslink layer is formed.

The formation of the surface crosslink layer may be conducted by a common method of increasing the crosslinking density of polymer particle surface, and for example, it may be conducted by mixing the ground polymer with a surface crosslinking solution comprising a surface crosslinking agent, and heat treating the mixture to subject it to a crosslink reaction.

The step 4 may be conducted at a temperature of about 80°C to about 250°C. More specifically, the surface crosslinking process may be conducted at a temperature of about 100°C to about 220°C, or about 110°C to about 200°C, or about 120°C to about 190°C, for about 10 minutes to about 2 hours, or about 20 minutes to about 60 minutes. If the crosslinking reaction temperature is less than 160°C or the reaction time is too short, a surface crosslinking reaction may not sufficiently occur, and thus, penetration degree may be lowered, and if the crosslinking reaction temperature is greater than 200°C or the reaction time is too long, water holding capacity may be lowered.

A temperature rise means for the surface crosslinking reaction is not specifically limited. A heat transfer medium may be supplied, or a heat source may be directly supplied for heating. Wherein, as the kind of heat transfer media that can be used, temperature-increased fluids such as steam, hot air, hot oil, and the like may be used, but the invention is not limited thereto, and the temperature of heat transfer medium supplied may be appropriately selected considering the means of heat transfer medium, temperature rise means, and target temperature. Meanwhile, as the heat source directly supplied, electric heating, and gas heating may be mentioned, but the present disclosure is not limited thereto.

Meanwhile, as explained above, the amino acetate-based chelating agent may be mixed after the step of heat treating the mixture to prepare super absorbent polymer in which a crosslink layer is formed on the surface of the base resin (step 4), which is favorable in that a possibility of generating tailings when treated with cysteine, and the like, after surface crosslinking, may be further lowered. Meanwhile, the mixing method of the amino acetate-based chelating agent is not specifically limited, and it may be mixed by simply mixing or spraying in the state of an aqueous solution.

Further, as explained above, the cysteine may be mixed after the step of heat treating the mixture to prepare super absorbent polymer in which a crosslink layer is formed on the surface of the base resin (step 4), which is favorable in that it exists on the surface of super absorbent polymer to increase contact area with odor, thereby improving deodorization capacity. Meanwhile, the mixing method of the cysteine is not specifically limited, and it may be mixed by spraying in the state of an aqueous solution or dry mixing.

Further, one or more additives selected from the group consisting of metal iodide salt, organic acid and polyphenol may be mixed after the step of heat treating the mixture to prepare super absorbent polymer in which a crosslink layer is formed on the surface of the base resin (step 4), which is favorable in that they exist on the surface of super absorbent polymer to increase contact area with odor, thereby improving deodorization capacity. Meanwhile, the mixing method of the additives is not specifically limited, and it may be mixed by spraying in the state of an aqueous solution or dry mixing.

According to one embodiment of the invention, after the step of preparing super absorbent polymer in which a crosslink layer is formed on the surface of the base resin (step 4), a step of mixing amino acetate-based chelating agent, cysteine, metal iodide salt, organic acid and polyphenol in the form of aqueous solutions (step of addition after hydration) may be included, and in this case, a drying step may be additionally conducted. To the drying process, the foregoing may be applied identically.

Hereinafter, preferable examples are presented to assist in understanding of the invention. However, the following examples are presented only as the illustrations of the invention, and are not intended to limit the invention.

**Examples and Comparative Examples: Preparation of super absorbent polymer**

**Example 1**

(Step 1) 100 g of acrylic acid, 0.37 g of N,N'-methylenebisacrylamide as a crosslinking agent, 0.15 g of sodium persulfate (SPS) as a thermal initiator, 0.008 g of benzoin ether as UV initiator, 40 g of caustic soda (NaOH), and 127 g of water were mixed, to prepare an aqueous monomer composition with a monomer concentration of 45.8 wt%. The aqueous monomer composition was introduced into a feed section of a polymerization reactor equipped with a continuously moving conveyor belt, and then, while maintaining a polymerization atmosphere temperature of 80$^{\circ}$C, UV was irradiated with a UV irradiation device (dose: 10 mW/cm$^2$), and UV polymerization was progressed for 2 minutes, thus preparing hydrogel polymer.

(Step 2) The hydrogel polymer was transferred to a meat chopper and cut to 2 mm to 10 mm. Wherein, the moisture content of cut hydrogel polymer was 47 wt%. Subsequently, the hydrogel polymer was dried in a hot air dryer of 170$^{\circ}$C for 30 minutes, and the dried hydrogel polymer was ground with a pin mill. Subsequently, polymers having particle sizes (average particle size) of 150 $\mu$m to 850 $\mu$m were classified with a sieve to prepare a base resin.

(Step 3) To 100 parts by weight of the above-prepared base resin, a surface crosslinking solution (2.5 parts by weight of water, 0.1 parts by weight of ethyleneglycol diglycidyl ether (EX-810), 0.1 parts by weight of aluminum sulfate 18 hydrate (Al-S), and 0.1 parts by weight of silica (Aerosil A200)) was uniformly mixed.

(Step 4) Next, a surface crosslinking reaction was progressed at 140$^{\circ}$C for 30 minutes. After completing the surface treatment, by classification with a sieve, super absorbent polymer with an average particle diameter of 150 to 850 $\mu$m was obtained. In the super absorbent polymer thus obtained, the content of polymer having average particle size less than 150 $\mu$m was less than 2%.

In the step 2, as the amino acetate-based chelating agent, EDTA was mixed by dissolving EDTA in an aqueous solution, and spraying it to the base resin, such that th e amount of EDTA is 0.5 parts by weight, based on 100 parts by weight of base resin.

Further, after the surface crosslinking of the step 4, L-cysteine was mixed by dissolving L-cysteine in an aqueous solution, and spraying it to the super absorbent polymer, such that the amount of L-cysteine is 0.2 parts by weight, based on 100 parts by weight of base resin. Thereafter, a drying step was conducted to prepare a composition of super absorbent polymer.

**Examples 2 to 17 and Comparative Examples 1 to 3**

Super absorbent polymer was prepared by the same method as Example 1, except that components, contents and addition times of the additives in Example 1 were changed as described in Table 1.

Meanwhile, in Examples 9 to 11, additional additives were sprayed in the form of aqueous solutions (metal iodide salt solution (1% concentration, CuI), tannin solution, citric acid solution) to the surface-crosslinked super absorbent polymer, and additional drying step was conducted.

[Table 1]

| | | Chelating agent | | | Amino acid | | | Other additives | | |
|---|---|---|---|---|---|---|---|---|---|---|
| | | Kind | Content | Addition time | Kind | Content | Addition time | Kind | Content | Addition time |
| | Example 1 | EDTA | 0.5 | Step 2 | L-cysteine | 0.2 | After step 4 | - | - | - |
| | Example 2 | EDTA | 0.1 | Step 2 | L-cysteine | 0.2 | After step 4 | - | - | - |
| | Example 3 | GLDA | 0.5 | Step 2 | L-cysteine | 0.2 | After step 4 | - | - | - |
| | Example 4 | GLDA | 1.0 | Step 2 | L-cysteine | 0.2 | After step 4 | - | - | - |
| | Example 5 | EDTA | 0.5 | Step 2 | L-cysteine | 0.1 | After step 4 | - | - | - |
| | Example 6 | EDTA | 0.5 | Step 2 | L-cysteine | 0.5 | After step 4 | - | - | - |
| | Example 7 | EDTA | 0.5 | Step 2 | L-cysteine | 1.5 | After step 4 | - | - | - |
| | Example 8 | EDTA | 0.5 | After step 4 | L-cysteine | 0.2 | After step 4 | - | - | - |
| | Example 9 | EDTA | 0.5 | Step 2 | L-cysteine | 0.2 | After step 4 | Metal iodide salt | 1.0 | After step 4 |
| | Example 10 | EDTA | 0.5 | Step 2 | L-cysteine | 0.2 | After step 4 | Tannin | 1.0 | After step 4 |
| | Example 11 | EDTA | 0.5 | Step 2 | L-cysteine | 0.2 | After step 4 | Citric acid | 1.0 | After step 4 |
| | Example 12 | EDTA | 0.25 | Step 2 | L-cysteine | 0.2 | After step 4 | - | - | - |
| | Example 13 | GLDA | 0.25 | Step 2 | L-cysteine | 0.2 | After step 4 | - | - | - |
| | Example 14 | EDTA | 0.25 | Step 2 | L-cysteine | 0.05 | After step 4 | - | - | - |
| | Example 15 | MGDA | 0.5 | Step 2 | L-cysteine | 0.2 | After step 4 | - | - | - |
| | Example 16 | HEDTA | 0.5 | Step 2 | L-cysteine | 0.2 | After step 4 | - | - | - |
| | Example 17 | EDG | 0.5 | Step 2 | L-cysteine | 0.2 | After step 4 | - | - | - |
| | Example 18 | DTPA | 0.5 | Step 2 | L-cysteine | 0.2 | After step 4 | - | - | - |
| | Comparative Example 1 | EDTA | - | Step 2 | - | 0.2 | After step 4 | - | - | - |
| | Comparative Example 2 | EDTA | 0.1 | Step 2 | - | 0.2 | After step 4 | - | - | - |

(continued)

|  | Chelating agent | | | Amino acid | | | Other additives | | |
|---|---|---|---|---|---|---|---|---|---|
|  | Kind | Content | Addition time | Kind | Content | Addition time | Kind | Content | Addition time |
| Comparative Example 3 | - | - | - | L-cysteine | 0.2 | After step 4 | - | - | - |
| Comparative Example 4 | Metal salt of recinoleic acid | 0.1 | Step 2 | L-cysteine | 0.2 | After step 4 | - | - | - |
| Comparative Example 5 | EDTA | 0.25 | Step 2 | Methionine | 0.2 | After step 4 | - | - | - |

**Experimental Example**

For the compositions of super absorbent polymers prepared in Examples and Comparative Examples, the properties were measured as follows.

**(1) Evaluation of absorption properties**

**1) Centrifuge Retention Capacity (CRC)**

Centrifuge retention capacity of each super absorbent polymer composition of Examples and Comparative Examples by absorption rate under no load was measured according to European Disposables and Nonwovens Association, (EDANA) standard EDANA WSP 241.3.

Specifically, from each super absorbent polymer composition obtained through Examples and Comparative Examples, polymers classified with sieves of #30-50 were obtained. $W_0$(g, about 0.2g) of such polymer was uniformly put in an envelope made of non-woven fabric, and the envelope was sealed, and then, dipped in a 0.9 wt% saline solution. After 30 minutes, the envelope was drained at 250G for 3 minutes using a centrifuge, and the weight $W_2$(g) of the envelope was measured. And, the same operation was conducted using an empty envelope without polymer, and then, the weight $W_1$(g) at that time was measured.

Using the weights thus obtained, CRC(g/g) was confirmed by the following Mathematical Formula 1.

$$[\text{Mathematical Formula 1}]$$

$$CRC\ (g/g) = \{[W_2(g) - W_1(g)]/W_0(g)\} - 1$$

**2) AUP: Absorbency under Pressure (AUP)**

For each super absorbent polymer composition of Examples and Comparative Examples, 0.7 psi absorbency under pressure was measured according to EDANA method WSP 242.3.

First, when measuring absorbency under pressure, the classified polymer used to measure CRC was used.

Specifically, on the bottom of a plastic cylinder having an inner diameter of 25 mm, a 400 mesh wire netting made of stainless steel was installed. And, $W_0$(g) of the super absorbent polymer was uniformly distributed on the screen under conditions of room temperature and 50% humidity, and a piston capable of uniformly applying a load of 0.7 psi was put thereon, wherein a piston having an outer diameter slightly smaller than 25 mm was used such that there was no gap with the inner wall of the cylinder and the up and down movement was not hindered. At this time, the weight $W_3$(g) of the apparatus was measured.

Inside a petri dish having a diameter of 150mm, a glass filter having a diameter of 90mm and a thickness of 5mm was laid, and a 0.9 wt% saline solution was poured to the same level as the upper side of the glass filter. And, one piece of a filter paper having a diameter of 90 mm was laid thereon. And then, the above measurement apparatus was put on the filter paper, and the solution was absorbed under pressure for 1 hour. After 1 hour, the measurement apparatus was lifted, and the weight $W_4(g)$ was measured. Using the obtained weights, absorbency under pressure(g/g) was calculated according to the following Mathematical Formula 2.

$$[\text{Mathematical Formula 2}]$$

$$AUP(g/g) = [W_4(g) - W_3(g)]/W_0(g)$$

**(2) Evaluation of bacteria inhibition rate**

Bacteria inhibition rate of each super absorbent polymer composition of Examples and Comparative Examples was evaluated as follows.

Specifically, commercial bacteria of E. coli (Escherichia Coli ATCC25922) were cultured in LB-Broth for 24 hours, and then, the cultured bacteria were diluted and injected in 2g of each sample of the super absorbent polymer composition together with artificial urine, and it was cultured at 37 ºC for 24 hours, and then, bacteria were extracted and the number of bacteria was measured (mean value of 2 specimens).

Bacteria inhibition rate (%) = (1-((bacteria amount of sample) / bacteria amount of (Reference sample (sample without deodorization capacity))) X 100(%)

The experiment results of the bacteria inhibition rate were shown in Table 2. The numerical values of Table 2 indicate bacteria inhibition rate compared to Comparative Example 1, and the higher the numerical value, the higher the bacteria inhibition rate.

**(3) Deodorization capacity (deodorization rate)**

Deodorization capacity was measured by an adsorption tube measurement method. Ketone (diacetyl), sulfur compound (dimethyl trisulfide) and aldehyde (3-methyl butanal) were selected as malodorous substances and deodorization capacity was tested.

- Adsorption tube measurement method : Into a 500mL glass bottle, 1 g of super absorbent polymer was put, and then, 25mL of malodorous substance was introduced. And then, aging was progressed in a constant temperature chamber for 3 hours, followed by capturing for 20 minutes. Wherein, the temperature of the constant temperature chamber was 35ºC, and N2 flow rate was 230mL/min. The odor pushed out was then adsorbed to the connected adsorption tube, which was repeated twice per the same sample to capture. The result of capture was confirmed by GC analysis.

-

Deodorization rate (%) = the amount of odor of Reference sample (sample without deodorization capacity) measured by GC - the amount of odor sample measured by GC / the amount of odor of Reference sample (sample without deodorization capacity) measured by GC X 100(%)

The experiment results of deodorization capacity were shown in Table 2. The numerical values of Table 2 indicate deodorization efficiencies compared to Comparative Example 1, and the higher the numerical value, the higher the deodorization efficiency.

[Table 2]

| | Bacteria inhibition rate (%) | Deodorization capacity | | | Absorption properties | |
|---|---|---|---|---|---|---|
| | | Aldehyde (%) | Ketone (%) | Sulfur compound (%) | CRC (g/g) | 0.7 AUP (g/g) |
| Example 1 | 99 | 90 | 90 | 85 | 31.0 | 22.0 |
| Example 2 | 99 | 94 | 94 | 87 | 30.5 | 21.7 |

(continued)

| | Bacteria inhibition rate (%) | Deodorization capacity | | | Absorption properties | |
|---|---|---|---|---|---|---|
| | | Aldehyde (%) | Ketone (%) | Sulfur compound (%) | CRC (g/g) | 0.7 AUP (g/g) |
| Example 3 | 99 | 95 | 92 | 83 | 31.2 | 22.0 |
| Example 4 | 99 | 90 | 90 | 85 | 31.0 | 22.3 |
| Example 5 | 99 | 90 | 90 | 82 | 31.7 | 21.7 |
| Example 6 | 99 | 90 | 90 | 93 | 31.2 | 22.2 |
| Example 7 | 99 | 90 | 90 | 98 | 30.1 | 20.2 |
| Example 8 | 99 | 90 | 90 | 93 | 31.0 | 22.0 |
| Example 9 | 99 | 94 | 93 | 98 | 30.2 | 22.1 |
| Example 10 | 99 | 98 | 97 | 98 | 30.7 | 21.5 |
| Example 11 | 99 | 95 | 93 | 90 | 30.0 | 20.8 |
| Example 12 | 90 | 75 | 67 | 83 | 31.0 | 22.2 |
| Example 13 | 98 | 88 | 85 | 85 | 31.6 | 21.9 |
| Example 14 | 85 | 75 | 67 | 67 | 32.0 | 21.8 |
| Example 15 | 99 | 92 | 90 | 85 | 30.1 | 20.2 |
| Example 16 | 99 | 90 | 90 | 80 | 31.2 | 22.0 |
| Example 17 | 99 | 90 | 90 | 85 | 30.2 | 21.1 |
| Example 18 | 99 | 98 | 97 | 83 | 30.1 | 21.5 |
| Comparati ve Example 1 | 0 | 0 | 0 | 0 | 32.3 | 22.5 |
| Comparati ve Example 2 | 85 | 0 | 0 | 0 | 32.0 | 22.7 |
| Comparati ve Example 3 | 0 | 90 | 85 | 82 | 31.0 | 21.5 |
| Comparati ve Example 4 | 35 | 75 | 80 | 78 | 30.5 | 20.5 |
| Comparati ve Example 5 | 98 | 97 | 63 | 45 | 30.0 | 21.0 |

From the results of Table 2, it can be confirmed that the compositions of super absorbent polymer according to Examples of the present disclosure, by using amino acetate-based chelating agent and cysteine in combination, exhibit excellent absorption properties, and simultaneously, have excellent deodorization capacity and odor masking capacity, and can effectively inhibit bacteria growth during wearing of a product.

It can be confirmed that in the case of Comparative Examples wherein only a part of the two additives was used, it was difficult to simultaneously realize bacteria inhibition rate and deodorization capacity. Particularly, in case a metal salt of ricinoleic acid was used instead of the amino acetate-based chelating agent in combination with cysteine, although deodorization capacity was similar to those of Examples, bacteria growth inhibition rate was remarkably low.

## Claims

1. A composition of super absorbent polymer comprising:

   a super absorbent polymer comprising a base resin comprising crosslinked polymer formed by crosslinking polymerization of acrylic acid-based monomers having at least partially neutralized acid groups and an internal crosslinking agent, and a surface crosslink layer formed on a surface of the base resin, in which the crosslinked polymer is additionally crosslinked by a surface crosslinking agent;
   an amino acetate-based chelating agent; and
   cysteine,
   wherein the amino acetate-based chelating agent and the cysteine are each independently included inside the surface crosslink layer or on a surface of the surface crosslink layer.

2. The composition of super absorbent polymer according to claim 1, wherein the amino acetate-based chelating agent includes one or more selected from the group consisting of ethylenediamine tetraacetic acid (EDTA), L-glutamic acid diacetate (GLDA), methyl glycine diacetic acid (MGDA), hydroxyethyl ethylenediamine triacetic acid (HEDTA), ethanol diglycinic acid (EDG), diethylenetriamine pentaacetic acid (DTPA), and salts thereof.

3. The composition of super absorbent polymer according to claim 1, wherein the amino acetate-based chelating agent is included in the content of 0.01 to 3 parts by weight, based on 100 parts by weight of the base resin.

4. The composition of super absorbent polymer according to claim 1, wherein the cysteine is included in the content of 0.01 to 3 parts by weight, based on 100 parts by weight of the base resin.

5. The composition of super absorbent polymer according to claim 1, further comprising one or more additives selected from the group consisting of metal iodide salt, organic acid and polyphenol.

6. A method for preparing a composition of super absorbent polymer, comprising steps of:

   step 1: subjecting acrylic acid-based monomers having at least partially neutralized acid groups to crosslinking polymerization, in the presence of an internal crosslinking agent and a polymerization initiator, to form a hydrogel polymer;
   step 2: preparing base resin comprising a crosslinked polymer obtained by drying and grinding the hydrogel polymer;
   step 3: mixing a surface crosslinking composition with the base resin to prepare a mixture; and
   step 4: heat treating the mixture to prepare the super absorbent polymer in which a surface crosslink layer is formed on the surface of the base resin,
   wherein an amino acetate-based chelating agent and cysteine are each independently mixed in at least one step of the steps 2 to 4 and steps before and after these steps.

7. The method for preparing a composition of super absorbent polymer according to claim 6, wherein the amino acetate-based chelating agent is mixed in the step 2, or mixed after the step 4.

8. The method for preparing a composition of super absorbent polymer according to claim 6, wherein the cysteine is mixed after the step 4.

9. The method for preparing a composition of super absorbent polymer according to claim 6, further comprising a step of mixing at least one additive selected from the group consisting of metal iodide salt, organic acid and polyphenol.

10. The method for preparing a composition of super absorbent polymer according to claim 9, wherein the additives are each independently mixed in at least one step of the steps 2 to 4 and steps before and after these steps.

11. The method for preparing a composition of super absorbent polymer according to claim 9, wherein the additives are each independently mixed after the step 4.

## INTERNATIONAL SEARCH REPORT

| International application No. |
| --- |
| **PCT/KR2023/003922** |

**A.     CLASSIFICATION OF SUBJECT MATTER**

**C08K 5/372**(2006.01)i; **C08K 5/20**(2006.01)i; **C08L 33/06**(2006.01)i; **C08J 3/24**(2006.01)i

According to International Patent Classification (IPC) or to both national classification and IPC

**B.     FIELDS SEARCHED**

Minimum documentation searched (classification system followed by classification symbols)

C08K 5/372(2006.01); A61F 13/15(2006.01); A61L 15/28(2006.01); A61L 15/60(2006.01); B01J 20/26(2006.01); C08F 2/44(2006.01); C08F 20/10(2006.01); C08F 220/10(2006.01); C08J 3/075(2006.01); C08L 101/14(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 고흡수성 수지(super absorbent polymer), 중합(polymerization), 가교제(cross-link ing agent), 킬레이트(chelate), 시스테인(cysteine)

**C.     DOCUMENTS CONSIDERED TO BE RELEVANT**

| Category* | Citation of document, with indication, where appropriate, of the relevant passages | Relevant to claim No. |
| --- | --- | --- |
| X | KR 10-2007-0104663 A (NIPPON SHOKUBAI CO., LTD.) 26 October 2007 (2007-10-26) See claims 1-13; and paragraphs [0037]-[0173]. | 1-11 |
| A | KR 10-0585441 B1 (THE PROCTER & GAMBLE COMPANY) 07 June 2006 (2006-06-07) See entire document. | 1-11 |
| A | KR 10-1812895 B1 (LOTTE CHEMICAL CORPORATION) 28 December 2017 (2017-12-28) See entire document. | 1-11 |
| A | JP 2009-515691 A (STOCKHAUSEN GMBH) 16 April 2009 (2009-04-16) See entire document. | 1-11 |
| A | KR 10-2015-0142636 A (LG CHEM, LTD.) 22 December 2015 (2015-12-22) See entire document. | 1-11 |

☐ Further documents are listed in the continuation of Box C.      ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **28 June 2023** | **28 June 2023** |

| Name and mailing address of the ISA/KR | Authorized officer |
| --- | --- |
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT
### Information on patent family members

International application No.

**PCT/KR2023/003922**

| Patent document cited in search report | | | Publication date (day/month/year) | Patent family member(s) | | | Publication date (day/month/year) |
|---|---|---|---|---|---|---|---|
| KR | 10-2007-0104663 | A | 26 October 2007 | AU | 2006-216015 | A1 | 24 August 2006 |
| | | | | CN | 101120038 | A | 06 February 2008 |
| | | | | CN | 101120038 | B | 14 December 2016 |
| | | | | CN | 104086938 | A | 08 October 2014 |
| | | | | CN | 104086938 | B | 19 September 2017 |
| | | | | EP | 1690887 | A1 | 16 August 2006 |
| | | | | EP | 1690887 | B1 | 21 May 2008 |
| | | | | EP | 1848758 | A1 | 31 October 2007 |
| | | | | EP | 1848758 | A4 | 30 November 2011 |
| | | | | EP | 1848758 | B1 | 07 November 2012 |
| | | | | EP | 1848758 | B2 | 17 February 2021 |
| | | | | JP | 2006-297373 | A | 02 November 2006 |
| | | | | JP | 2006-299234 | A | 02 November 2006 |
| | | | | JP | 2012-086221 | A | 10 May 2012 |
| | | | | JP | 2014-039927 | A | 06 March 2014 |
| | | | | JP | 2016-033224 | A | 10 March 2016 |
| | | | | JP | 4965865 | B2 | 04 July 2012 |
| | | | | JP | 5046522 | B2 | 10 October 2012 |
| | | | | JP | 5625003 | B2 | 12 November 2014 |
| | | | | JP | 5903086 | B2 | 13 April 2016 |
| | | | | JP | 6025953 | B2 | 16 November 2016 |
| | | | | US | 2006-0183828 | A1 | 17 August 2006 |
| | | | | US | 2010-0062252 | A1 | 11 March 2010 |
| | | | | US | 8182916 | B2 | 22 May 2012 |
| | | | | WO | 2006-088115 | A1 | 24 August 2006 |
| KR | 10-0585441 | B1 | 07 June 2006 | CN | 100247268 | C | 29 March 2006 |
| | | | | CN | 100437487 | A | 20 August 2003 |
| | | | | EP | 1149595 | A1 | 31 October 2001 |
| | | | | EP | 1276513 | A1 | 22 January 2003 |
| | | | | EP | 1276513 | B1 | 27 November 2013 |
| | | | | JP | 2003-530968 | A | 21 October 2003 |
| | | | | JP | 4738699 | B2 | 03 August 2011 |
| | | | | KR | 10-2002-0093067 | A | 12 December 2002 |
| | | | | US | 6960655 | B2 | 01 November 2005 |
| KR | 10-1812895 | B1 | 28 December 2017 | | None | | |
| JP | 2009-515691 | A | 16 April 2009 | EP | 1957193 | A2 | 20 August 2008 |
| | | | | EP | 1957193 | B1 | 03 October 2012 |
| | | | | JP | 2013-163818 | A | 22 August 2013 |
| | | | | KR | 10-1407781 | B1 | 20 June 2014 |
| | | | | KR | 10-2008-0077630 | A | 25 August 2008 |
| | | | | US | 2010-0035757 | A1 | 11 February 2010 |
| | | | | US | 8653320 | B2 | 18 February 2014 |
| | | | | WO | 2007-057203 | A2 | 24 May 2007 |
| | | | | WO | 2007-057203 | A3 | 13 December 2007 |
| KR | 10-2015-0142636 | A | 22 December 2015 | JP | 2017-517600 | A | 29 June 2017 |
| | | | | JP | 6592461 | B2 | 16 October 2019 |
| | | | | KR | 10-1743274 | B1 | 02 June 2017 |
| | | | | US | 2017-0189575 | A1 | 06 July 2017 |
| | | | | US | 2019-0167836 | A1 | 06 June 2019 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020220039062

- KR 1020230038011

**Non-patent literature cited in the description**

- **REINHOLD SCHWALM.** UV Coatings: Basics, Recent Developments and New Application. Elsevier, 2007, 115

- **ODIAN.** Principle of Polymerization. Wiley, 1981, 203